(19) Europäisches Patentamt
European Patent Office
Office européen des brevets

(11) Veröffentlichungsnummer: **0 208 971 B1**

(12) **EUROPÄISCHE PATENTSCHRIFT**

(45) Veröffentlichungstag der Patentschrift: 29.05.91

(51) Int. Cl.⁵: **A61K 31/71**, A61K 9/30, A61K 47/02

(21) Anmeldenummer: 86108791.4

(22) Anmeldetag: 27.06.86

Die Akte enthält technische Angaben, die nach dem Eingang der Anmeldung eingereicht wurden und die nicht in dieser Patentschrift enthalten sind.

(54) Feste Arzneimittelformen zur peroralen Anwendung enthaltend 9-Deoxo-11-deoxy-9,11-(imino(2-(2-methoxyethoxy)ethyliden)-oxy)-(9S)-erythromycin und Verfahren zu ihrer Herstellung.

(30) Priorität: 10.07.85 DE 3524572

(43) Veröffentlichungstag der Anmeldung: 21.01.87 Patentblatt 87/04

(45) Bekanntmachung des Hinweises auf die Patenterteilung: 29.05.91 Patentblatt 91/22

(84) Benannte Vertragsstaaten: AT BE CH DE FR GB IT LI LU NL SE

(56) Entgegenhaltungen:
GB-A- 1 520 963    GB-A- 2 132 086
US-A- 3 081 233    US-A- 3 891 755
US-A- 4 000 254    US-A- 4 340 582

(73) Patentinhaber: **Dr. Karl Thomae GmbH**
**Postfach 1755**
**W-7950 Biberach (Riss)(DE)**

(72) Erfinder: **Etienne, Alain**
**Residence Orient 19 Allée du Pasten**
**F-33160 St.Medard en Jalles(FR)**
Erfinder: **Gruber, Peter, Dr.Dipl.-Chem.**
**Friedrich-Ebert-Strasse 70**
**W-7950 Biberach 1(DE)**
Erfinder: **Busch, Ulrich, Dr. Dipl.-Chem.**
**Köhlesrain 84/4**
**W-7950 Biberach 1(DE)**

**Beschreibung**

Die Erfindung betrifft neue orale, feste Arzneimittelformen enthaltend 9-Deoxo-11-deoxy-9,11-[imino[2-(2-methoxyethoxy)-ethyliden]oxy]-(9S)-erythromycin, im Folgenden AS-E 136 genannt, und Verfahren zu ihrer Herstellung.

In der DE-A1-2.515.075 (vgl. auch GB-A-1 520 963) wird AS-E 136 als eine besonders gut antibakteriell wirkende Substanz beschrieben. Eine gute antibakterielle Wirkung wird vor allen Dingen dann erzielt, wenn diese Substanz in gelöster Form unter Umgehung des Magen-Darmtraktes, z.B. intravenös, appliziert wird. AS-E 136 zeigt gegenüber anderen Makrolid-Antibiotika wesentliche Vorteile, da es nur 1-mal pro Tag mit einer Gesamtdosis bis 500 mg appliziert werden muß; es zeichnet sich im Vergleich zu anderen Antibiotika gleichen Strukturtyps, z.B. gegenüber Erythromycin, durch ungewöhnlich hohe und langanhaltende Gewebespiegel aus.

In üblicher Weise hergestellte oral zu applizierende Formen, die die Substanz AS-E 136 enthalten, führen jedoch nach ihrer Applikation nur zu sehr geringen und meistens schwankenden Gewebespiegelwerten, solche Formen haben sich deshalb bisher für die Applikation dieser Wirkstubstanz als ungeeignet erwiesen. Es bestand ein dringendes Bedürfnis, oral zu applizierende Arzneimittelformen, die AS-E 136 enthalten, und diese Nachteile nicht besitzen, aufzufinden bzw. zu entwickeln.

AS-E 136 ist charakterisiert durch eine ausgeprägte Empfindlichkeit in sauren Medien, die Wirksubstanz wird, wie für viele Makrolid-Antibiotika, wie Erythromycin, üblich, bei der Einwirkung von Magensaft (pH 1,0 bis 2,0) in kurzer Zeit zerstört. Das Hauptzersetzungsprodukt des As-E 136 ist Erythromycylamin, das zwar auch eine antibakterielle Aktivität entfaltet, aber bei oraler Applikation am Menschen in einem nur so geringen Ausmaß resorbiert wird, daß mikrobiologisch wirksame Blutspiegel mit dieser Substanz auch bei sehr hohen Dosierungen nicht oder nur unvollständig aufgebaut werden können. Dies läßt sich anhand der erzielten Werte für die die Flächen unter der Plasmaspiegelkurve (AUC - "area under the curve") verdeutlichen.

Tabelle 1 zeigt die AUC-Werte nach Gabe von Erythromycylamin

## Tabelle 1

AUC-Werte (gerundet) von Plasmaspiegeln nach peroraler Gabe als Tablette an 7 Probanden.

| Proband | AUC bis 24 Stunden post applic. $(\mu\,g/ml \times h) \times 1000$ |
|---|---|
| 1 | 700 |
| 2 | 300 |
| 3 | 500 |
| 4 | 500 |
| 5 | 550 |
| 6 | 100 |
| 7 | 250 |

Selbst bei pH-Werten um 5,5, bei denen Erythromycin für eine Resorption bereits ausreichend lang chemisch stabil ist, hält bei AS-E 136 die Hydrolyseempfindlichkeit noch unverändert an. Erst bei pH-Werten um oder über 7,0 gewinnt AS-E 136 an Stabilität, wie sich auch aus den nachfolgenden Tabellen 2 und 3 ergibt:

Tabelle 2

| pH-Wert | % intakte Erythromycin-Base | |
|---------|-----------------------------|----------------|
|         | nach 30 Min. | nach 60 Min. |
| 1,3 | 30 | 10 |
| 4,5 | 55 | 50 |
| 5,5 | 100 | 85 |

Tabelle 3

| pH-Wert | % intakte Substanz AS-E 136 |
|---------|------------------------------|
|         | nach 30 Minuten |
| 1,0 | 10 |
| 6,0 | 20 |
| 6,5 | 20 |
| 7,0 | 50 |
| 7,5 | 80 |
| 8,0 | 95 |

Man kann AS-E 136, gegebenenfalls mit weiteren Hilfsstoffen, wie Trägerstoffe und Sprengmittel, auch nicht einfach mit einem magensaftresistenten Lack, der den Wirkstoff erst bei pH-Werten über 7,0 freigibt, umhüllen, da ein derartiger pH-Bereich in dem zur Resorption zur Verfügung stehenden Magendarmtrakt nicht vorkommt. Dies ergibt sich bereits aus folgenden Überlegungen: Die durchschnittlichen pH-Werte des menschlichen Darmes liegen zwischen 5,0 und 7,0 und zwar 5,0 im obersten Darmabschnitt (Duodenum) und 7,0 im untersten Abschnitt (Colon). Weiterhin ist bekannt, daß das Ausmaß der Resorption in tieferen Darmabschnitten stark eingeschränkt ist. Dies gilt insbesondere für große Moleküle, wie das Makrolid AS-E 136. Es wäre zum Beispiel für einen magensaftlabilen Wirkstoff wie Erythromycin nicht schwierig, eine zuverlässige Arzneiform zu entwickeln, da der Wirkstoff im obersten Darmabschnitt (pH ca. 5 - 5,5) bereits ausreichend stabil ist. Es gehört in diesem Fall zum Stand des Wissens, den Wirkstoff, zum Beispiel mit geeigneten Hilfsstoffen, zu einer Tablette zu verpressen und diese mit sogenannten magensaftresistenten Lacken, wie Celluloseacetatphthalat oder Hydroxypropylmethylcellulosephthalat, zu überziehen. Nach Verlassen des Magens löst sich im Darmsaft dieser Lack ab, der Wirkstoff wird gelöst und resorbiert. Dieses bekannte Prinzip ist im Falle von AS-E 136 nicht anwendbar, weil der Wirkstoff, wie aus der Tabelle 2 ersichtlich, erst im pH-Bereich von 7,5 ausreichende Stabilität aufweist. Dieser pH-Wert wird in der Regel jedoch im Darm nicht erreicht bzw. nur im Bereich des Colon transcendens bzw. descendens. Hier aber ist die Arzneiform in den eingedickten Faeces eingeschlossen und das Resorptionsausmaß nur mehr sehr gering.

Die komplexe Abhängigkeit der AS-E 136 Resorption vom pH-Profil in den einzelnen Darmabschnitten und dem aus anderen Gründen abnehmenden Resorptionsausmaß in tieferen Darmbereichen kann mit AS-E-Formen, die aufgrund des gewählten magensaftresistenten Lackes den Wirkstoff in verschiedenen Darmbereichen abgeben, genau gezeigt werden. (siehe Tabelle 4).

Tabelle 4

AUC-Werte (gerundet) von Plasmaspiegeln von verschiedenen Chargen mit Wirkstoff-Freigabe bei unterschiedlichen pH-Werten

| Chargen-Nr. | Freigabe pH-Wert | AUC-Werte 0-24 Stunden (µg/ml x h) x 1000 | | |
|---|---|---|---|---|
| | | min. | | max. |
| ZP 234/12 | 5.0 | 1.0 | – | 56.0 |
| ZP 234/13 | 6.2-6.7 | 650.0 | – | 1650.0 |
| ZP 234/14 | 7.0-7.4 | 130.0 | – | 390.0 |

Die Charge ZP 234/12 besteht aus 23,52% Starterkerne aus 70% Saccharose und 30% Maisstärkepuder, 46,37% Wirkstoff, 15,65% Talk, 5,65% Polyvinylpyrrolidon, 7.05% Hydroxypropylmethylcellulosephthalat und 1,96% Rizinusöl; die Charge ZP 234/13 aus 24,91% Starterkerne derselben Zusammensetzung, 49,54% Wirksubstanz, 11,52% Talk, 6,05% Polyvinylpyrrolidon, 4,36% Eudragit S®, 1, 09% Eudragit L®, 1,64% Rizinusöl, 0,91% Magnesiumstearat; die Charge ZP 234/14 aus 23,5% Starterkerne derselben Zusammensetzung, 46,74% Wirkstoff, 11,60% Talkum, 5,71% Polyvinylpyrrolidon, 7,89% Eudragit S®, 1,03% Eudragit L®, 2,67% Rizinusöl, 0,86% Magnesiumstearat (die Prozente sind als Gewichtsprozente angegeben, Wirkstoff ist AS-E 136).

Aus obiger Tabelle ist ersichtlich, daß Pellets, die mit einem Lack überzogen sind, der die Freigabe des Wirkstoffs bei pH 5,0 erlaubt (pH-Bereich im Duodenum am Magenausgang) praktisch keine Blutspiegel an antimikrobieller Wirkung aufweisen. Wählt man einen Überzug für die gleichen Pellets, der eine Freigabe des Wirkstoffs bei einem pH-Wert zwischen 6,2 und 6,7 gestattet (Bereich des unteren Duodenum bis Ileum), so erzielt man damit nur schwankende und niedrige Blutspiegel, überzieht man aber diese Pellets mit einem Film, der die Freigabe des Wirkstoffs erst bei einem pH-Wert von 7,0 bis 7,4 erlaubt, so beobachtet man wiederum ein deutliches Absinken der Blutspiegel. Wird also ein Lack verwendet, der den Wirkstoff in einem Darmbereich mit pH 5,0 freigibt, so tritt praktisch kein Blutspiegel auf, d. h. der Wirkstoff wird rascher zersetzt als resorbiert. Setzt man dagegen den Wirkstoff in einem pH-Bereich frei, in welchem er stabiler ist (pH 7,0 bis 7,4), erzielt man praktisch ebenfalls keinen Blutspiegel, da in diesem Darmbereich (Colon) fast keine Resorption erfolgt. Bei einer Freisetzung des Wirkstoffs durch die Verwendung eines Lacks, der ihn in einem Darmbereich von pH-Werten zwischen 6,2 bis 6,7 freigibt, erreicht man nur sehr mäßige Blutspiegelwerte, da, wie aus der Tabelle 3 ersichtlich ist, die Geschwindigkeit der Wirkstoffzersetzung größer ist als die der Wirkstoffresorption. Zusammenfassend kann gesagt werden, daß sich bei Einsatz der verfügbaren, verschiedenartigsten magensaftresistenten Lacken keine geeignete feste Arzneiform mit zuverlässiger Resorption für AS-E 136 herstellen läßt.

Es wurde nun gefunden, daß sich feste, oral applizierbare Arzneimittelformen, die AS-E 136 enthalten und hohe und gering streuende Blutspiegelwerte garantieren, dann herstellen lassen, wenn man den feinverteilten Wirkstoff innig mit einem basischen Hilfsstoff in einem Verhältnis von 1 Mol Wirkstoff zu mindestens 2 basischer Hilfsstoff vermischt. Die Mischung kann einmal granuliert und gegebenenfalls nach Hinzufügung weiterer Hilfsstoffe, wie Sprengmittel, Bindemittel, Schmierstoffe, zu Tabletten verpresst werden oder die Mischung wird, gegebenenfalls nach Hinzufügung von Klebstoffen, pelletiert, z.B. durch Auftragen auf Zuckerkügelchen. Außerdem kann die Mischung zur Herstellung von Granulaten, gegebenenfalls nach Hinzufügen weiterer Hilfsstoffe, brikettiert werden, z.B. mittels eines Walzenkompaktors. Weiter kann die Mischung in einer Schmelze von Fetten, wie Stearylalkoholen, oder von Polyethylenglykolen, suspendiert und in einem Kühlturm zu kleinen, gleichmäßigen Teilchen sprüherstarrt werden. Die nach diesen Verfahren erhaltenen Formen, wie Tabletten, Pellets, Granulate, werden anschließend mit einem magensaftresistenten Lack, der in dem pH-Bereich zwischen 5,5 und 6,8 löslich ist bzw. der den Wirkstoff bei einem pH-Wert zwischen 5,5 und 6,8, vorzugsweise 6,0 und 6,4, freigibt, überzogen.

Ein Beispiel für ein erfindungsgemäßes Produkt zeigt Tabelle 5, auf der die AUC-WErte von Plasmaspiegeln nach Gabe einer Mischung von 100 mg AS-E 136 und 100 mg Magnesiumcarbonat gemäß Beispiel 1 aufgelistet werden.

## Tabelle 5

AUC-Werte (gerundet) von Plasmaspiegeln nach p.o-Gabe von 500 mg AS-E 136 als Tablette an 12 Probanden

| Proband | AUC bis 24 Stunden p.a. (μg/ml x h) x 1000 |
|---|---|
| 1 | 2 600 |
| 2 | 1 900 |
| 3 | 3 800 |
| 4 | 1 900 |
| 5 | 2 100 |
| 6 | 2 800 |
| 7 | 1 500 |
| 8 | 1 000 |
| 9 | 2 800 |
| 10 | 2 600 |
| 11 | 2 600 |
| 12 | 3 800 |

Als basische Hilfsstoffe dienen, für sich oder in Kombination miteinander Oxide, Hydroxide oder Carbonate von Magnesium oder Calcium, wie Magnesiumoxid, Magnesiumhydroxid, Magnesiumcarbonat, Magnesiumhydrogencarbonat, Calciumhydroxid, Calciumcarbonat, Magnesiumaluminiumhydroxid, Magnesiumaluminat, oder Carbonate, Hydrogencarbonate oder Hydroxide von Natrium oder Kalium, wie Natriumcarbonat, Natriumhydrogencarbonat, Kaliumcarbonat, Kaliumhydrogencarbonat, Natrium-oder Kaliumhydroxid, aber auch stark basische Alkalisalze, wie Trinatriumphosphat. Bevorzugte basische Hilfsstoffe sind Magnesiumcarbonat, Magnesiumoxid und Natriumcarbonat.

Der Wirkstoff und der basische Hilfsstoff sind in einem Verhältnis von mindestens 1 mol Wirkstoff zu 2 Val basischer Hilfsstoff zu vermischen. Eine obere Mengenbegrenzung des basischen Hilfsstoffes ergibt sich aus der Größe der dabei resultierenden Arzneiformen und/oder aus der physiologischen Verträglichkeit. des basischen Hilfsstoffes. Eine volumenmäßig große Arzneimittelform läßt sich schlecht applizieren. Im allgemeinen liegt die obere Grenze des basischen Hilfsstoffes bei 200 Val. Das bevorzugte Verhältnis von Wirkstoff zu basischem Hilfsstoff beträgt 1 mol Wirkstoff zu 15 bis 50 Val basischer Hilfsstoff.

Als magensaftresistente Lacke dienen vorzugsweise Hydroxypropylmethylcellulosephthalat, Copolymerisate aus Methacrylsäure und -methylester (Eudragit L[R] und Eudragit S[R]), Celluloseacetatphthalat, Gemische dieser Lacke, aber auch sonstige Lacke, die sich im Bereich von pH 5,5 bis 6,8 auflösen. Die Menge des Lackes wird so gewählt, daß die mit dem entsprechenden Lack überzogenen Formen zwischen 30 Minuten und 2 Stunden magensaftresistent sind; kleine Teile, z.B. Granulate, Pellets, sollten mindestens 30 Minuten, größere Tabletten aber 2 Stunden magensaftresistent sein. Magensaftresistent bedeutet, diese Formen sollten innerhalb der oben genannten Zeiten praktisch nicht den Wirkstoff bei einem Freigabetest mit künstlichem Magensaft nach außen abgeben.

Der Zusatz eines Sprengmittels bringt vor allem bei volumenmäßig großen Arzneimittelformen eine rasche Freisetzung des mit dem basischen Hilfsmittel versehenen Wirkstoffes und sorgt so für rasch einsetzende Resorption und Wirkung. Das Sprengmittel sorgt z.B. bei Tabletten für den raschen Zerfall in Granulatkörner. Dadurch wird die Oberfläche der Arzneiformmasse vergrößert, der basische Hilfsstoff schützt den säurelabilen Wirkstoff über einen genügenden Zeitraum, innerhalb dessen der Wirkstoff zur Resorption kommt. Die Menge des Sprengmittels wird in Abhängigkeit von dessen Aktivität so bemessen, daß der Arzneiformkern, z.B. Tablettenkern, innerhalb einer Stunde, bevorzugt innerhalb von 30 Minuten zerfällt. Als Sprengmittel kommen die hierfür üblicherweise verwendeten Hilfsstoffe, wie Stärke, Croscarmelose-Natrium (AC-DT-SOL$^{(R)}$), Natrium-Stärkeglycolat oder Crospovidone, allein oder in Kombination miteinander in Frage.

Es ist wichtig für alle erfindungsgemäßen Arzneiformen, daß der bis zu pH-Werten von 7 empfindliche Wirkstoff immer in enger Verbindung mit dem basischen Hilfsstoff vorliegt. Dies wird durch das innige Vermischen beider Substanzen und, beispielsweise, durch das Verpressen dieses Gemisches, gegebenenfalls nach Hinzufügung weiterer Hilfsstoffe, zu Tabletten erreicht. Als weitere Hilfsstoffe sind besonders die oben geschilderten Sprengmittel, aber auch sonst übliche Zusatzstoffe, wie Polyvinylpyrrolidon, oder Schmiermittel, wie Magnesiumstearat gemeint. Das zu tablettierende Gut wird befeuchtet, granuliert und das getrocknete Granulat zu Tabletten verpreßt. Eine solche Tablette wird erfindungsgemäß mit einem oben genanten magensaftresistenten Lack überzogen, beispielsweise durch Besprühen mit einer geeigneten Lacklösung.

Eine andere Möglichkeit besteht darin, das innige Gemisch aus Wirkstoff und basischem Hilfsstoff mit einem Klebstoff, wie Polyvinylpyrrolidon, zu versehen und dieses Gemisch auf Pellet-Starterkerne "aufzukleben". Als Pellet-Starterkerne dienen z.B. ausgerundete Zuckerkügelchen; die Suspension aus Wirkstoff, basischem Hilfsstoff und Klebstoff wird auf diese Pellet-Starterkerne aufgesprüht. Zur Pelletierung des oben genannten innigen Gemisches eignen sich auch viele andere, an sich bekannte Methoden.

Will man dagegen ein Wirkstoffgranulat herstellen, so kann man auch ein inniges Gemisch aus Wirkstoff, basischem Hilfsstoff und weiteren Zusatzstoffen, z.B. Klebstoffen wie Polyvinylpyrrolidon, in angefeuchtetem Zustand über einen Walzenkompaktor brikettieren. Die dabei erzeugten groben Teilchen werden aufgebrochen, die geeignete Fraktion wird abgesiebt und der Rest nochmals an den Walzenkompaktor zurückgegeben.

Will man besonders kleine und gleichmäßig große Teilchen erzeugen, z.B. für die Herstellung von Arzneimittelsuspensionen, wobei die Teilchen dann bevorzugt einen Durchmesser von 0,2 bis 0,5 mm aufweisen sollten, so bedient man sich vorteilhafterweise der Sprüherstarrung. In diesem Fall wird der Wirkstoff mit dem basischem Hilfsstoff in einer Schmelze suspendiert und diese Schmelze in einem Kühlturm versprüht. Als Schmelze eignen sich z.B. Fette bzw. Fettalkohole mit einem scharfen Schmelzpunkt, z.B. Stearylalkohol, aber auch andere Materialien mit ähnlichen physikalischen Eigenschaften, z.B. Polyethylenglykole.

Alle nach diesen Verfahren erhaltenen Teilchen werden jeweils anschließend mit einem magensaftresistenten Lack, der sich in einem pH-Bereich zwischen 5,5 und 6,8 löst, überzogen.

Nach dem Ablösen des magensaftresistenten Lackes im Darmtrakt bildet sich um den Wirkstoff eine basische Mikrosphäre mit einem gegenüber der Umgebung erhöhten pH-Wert, der den Wirkstoff in diesem Bereich stabilisiert. Die vorliegenden Teilchen mit ihren Mikrosphären bewegen sich bevorzugt entlang der Darmschleimhaut. Aufgrund des dadurch resultierenden kurzen Diffusionsweges wird es ermöglicht, daß der Wirkstoff weitgehend unzersetzt resorbiert wird, wobei dem Wirkstoff zugute kommt, daß er eine verhältnismäßig hohe Resorptionsrate aufweist im Gegensatz zu Erythromycylamin, seinem Zersetzungsprodukt.

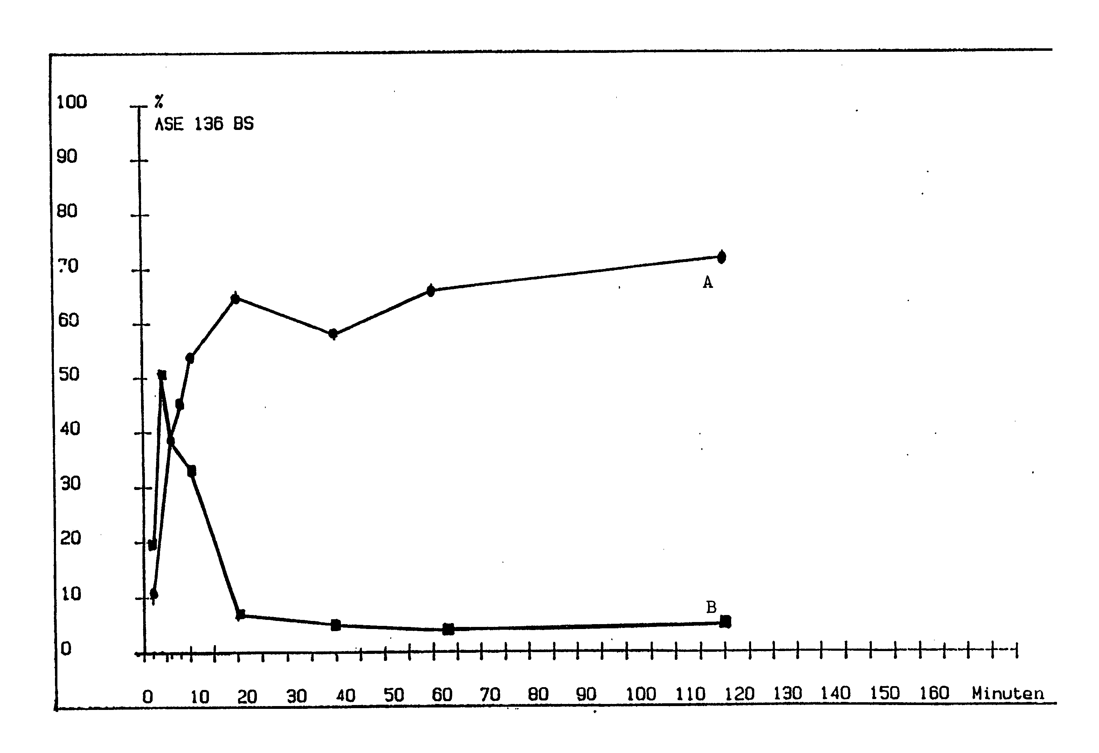

Die Zeichnung zeigt das Auflösungsprofil einer gemäß Beispiel 1 hergestellten Tablette in einem künstlichen Darmsaft von pH 6,0 im Vergleich zu einer Tablette ohne basischen Hilfsstoff. Die Tablettenkerne wurden jeweils in eine USPXX-Apparatur gegeben und bei 100 U/min. mit dem Paddle gerührt. In der Figur ist auf der Ordinate das unzersetzte AS-E 136 in Abhängigkeit von der Zeit dargestellt. Die Kurve A entspricht der gemäß Beispiel 1 hergestellten Tablette, Kurve B der Vergleichstablette ohne Zusatz basischer Hilfsstoffe.

Die nachfolgenden Beispiele sollen das Wesen der Erfindung erläutern:

Beispiel 1

Zusammensetzung einer Tablette:

| AS-E 136 | 100 | mg | (1) |
| Magnesiumcarbonat | 100 | mg | (2) |
| Polyvinylpyrrolidon, quervernetzt | 44.5 | mg | (3) |
| Polyvinylpyrrolidon | 5,0 | mg | (4) |
| Magnesiumstearat | 1,0 | mg | (5) |
| Celluloseacetatphthalat | 24,8 | mg | (6) |
| Dibutylphthalat | 1,2 | mg | (7) |
| | 276,5 | mg | |

In dem angegebenen Verhältnis werden der Wirkstoff (1), der basische Hilfstoff (2) und das Sprengmittel (3) gemischt und mit dem Klebstoff (4), gelöst in Isopropanol, befeuchtet, granuliert und getrocknet. Nach Zumischung des Schmiermittels (5) wird das Material zu Tabletten verpreßt, die mit einem Gemisch der Lackkomponenten (6) und (7), gelöst in Äthanol/Methylenchlorid, durch Besprühen überzogen werden.

Die magensaftresistente Tablette wird in einer US P XX-Freigabeapparatur getestet (Paddle-Methode, 100 U/Min., 37° C). Die gleiche Form wird nacheinander in den einzelnen Medien gerührt.

| Zeit | Medium | pH-Wert | Freigabe in % |
| --- | --- | --- | --- |
| 1 Stunde | künstl. Magensaft | 1,2 | 0 |
| 1 Stunde | künstl. Darmsaft | 4,5 | 0 |
| 15 Min. | künstl. Darmsaft | 6,0 | 21,1 |
| 30 Min. | künstl. Darmsaft | 6,0 | 98,4 |

Beispiel 2

Zusammensetzung einer Tablette:

| AS-E 136 | 100 | mg | (1) |
| Magnesiumcarbonat | 250 | mg | (2) |
| Sprengmittel Stärke | 44,5 | mg | (3) |
| Polyvinylpyrrolidon | 8,0 | mg | (4) |
| Magnesiumstearat | 1,0 | mg | (5) |
| Celluloseacetatphthalat | 27,7 | mg | (6) |
| Dibutylphthalat | 1,4 | mg | (7) |
| | 432,6 | mg | |

Wie unter Beispiel 1 angegeben, werden Tabletten mit dieser Zusammensetzung hergestellt. Freigabewerde in Abhängigkeit von Zeit und pH-Wert:

| Zeit | Medium | pH-Wert | Freigabe in % |
|------|--------|---------|---------------|
| 1 Stunde | künstl. Magensaft | 1,2 | 0 |
| 1 Stunde | künstl. Darmsaft | 4,5 | 0 |
| 15 Min. | künstl. Darmsaft | 6,0 | 10,9 |
| 30 Min. | künstl. Darmsaft | 6,0 | 99,2 |

Beispiel 3

Zusammensetzung einer Tablette:

| | | | |
|------|------|------|-----|
| AS-E 136 | 100 | mg | (1) |
| Magnesiumoxid | 175 | mg | (2) |
| Sprengmittel Stärke | 65 | mg | (3) |
| Mikrokristalline Cellulose | 110 | mg | (4) |
| Polyvinylpyrrolidon | 2 | mg | (4) |
| Magnesiumstearat | 2,5 | mg | (5) |
| Celluloseacetatphthalat | 31,8 | mg | (6) |
| Dibutylphthalat | 1,6 | mg | (7) |
| | 482,9 | mg | |

Wie unter Beispiel 1 angegeben, werden nach dieser Zusammensetzung magensaftresistente Tabletten hergestellt. Freigabewerte in Abhängigkeit von Zeit und pH-Wert:

| Zeit | Medium | pH-Wert | Freigabe in % |
|------|--------|---------|---------------|
| 1 Stunde | künstl. Magensaft | 1,2 | 0 |
| 1 Stunde | künstl. Darmsaft | 4,5 | 0 |
| 15 Min. | künstl. Darmsaft | 6,0 | 0 |
| 20 Min. | künstl. Darmsaft | 6,0 | 77,9 |
| 30 Min. | künstl. Darmsaft | 6,0 | 99,4 |

Beispiel 4

Zusammensetzung einer Tablette:

8

| | | |
|---|---|---|
| AS-E 136 | 125 mg | (1) |
| Aluminiumhydroxid | 20 mg | (2) |
| Calciumhydroxid | 10 mg | (2) |
| Sprengmittel Stärke | 35 mg | (3) |
| Polyvinylpyrrolidon | 6 mg | (4) |
| Magnesiumstearat | 1 mg | (5) |
| Copolymerisat Methacryl-säure-ester (Eudragit L) | 18 mg | (6) |
| Triacetin | 2 mg | (7) |
| | 217 mg | |

Wie unter Beispiel 1 angegeben, werden nach dieser Zusammensetzung magensaftresistente Tabletten hergestellt. Freigabewerte in Abhängigkeit von Zeit und pH-Wert:

| Zeit | Medium | pH-Wert | Freigabe in % |
|---|---|---|---|
| 2 Stunden | künstl. Magensaft | 1,2 | 0 |
| 15 Min. | künstl. Darmsaft | 6,2 | 32 |
| 30 Min. | künstl. Darmsaft | 6,2 | 96,4 |

Beispiel 5

Zusammensetzung einer Tablette:

| | | | |
|---|---|---|---|
| AS-E 136 | 125 | mg | (1) |
| Natriumcarbonat | 125 | mg | (2) |
| Sprengmittel Stärke | 56 | mg | (3) |
| Polyvinylpyrrolidon | 6 | mg | (4) |
| Magnesiumstearat | 3 | mg | (5) |
| Celluloseacetatphthalat | 30 | mg | (6) |
| Dibutylphthalat | 1,6 | mg | (7) |
| | 346.6 | mg | |

Wie unter Beispiel 1 angegeben, werden nach dieser Zusammensetzung magensaftresistente Tabletten hergestellt. Freigabewerte in Abhängigkeit von Zeit und pH-Wert:

| Zeit | Medium | pH-Wert | Freigabe in % |
|------|--------|---------|---------------|
| 2 Stunden | künstl. Magensaft | 1,2 | 0 |
| 15 Min. | künstl. Darmsaft | 6,0 | 24,3 |
| 30 Min. | künstl. Darmsaft | 6,0 | 98,2 |

Beispiel 6

AS-E 136 enthaltende Pellets:

| | | |
|------|------|-----|
| AS-E 136 | 2,8 kg | (1) |
| Calciumhydroxid | 4,2 kg | (2) |
| Sprengmittel Stärke | 1,2 kg | (3) |
| Methylcellulose | 0,4 kg | (4) |
| Polyäthylenglykol 6000 | 1,4 kg | (5) |
| Hydroxypropylmethyl-cellulosephthalat | 0,8 kg | (6) |
| Rizinusöl | 0,2 kg | (7) |
| Talcum | 0,4 kg | (8) |

Die Komponenten (1) bis (5) werden gemischt und mit Wasser befeuchtet. Die befeuchtete und gesiebte Masse extrudiert man durch ein Sieb von 0,8 mm. Die Stränge werden in einem Merumerizer gerundet. Die Komponenten (6) bis (8) werden in Isopropanol/Aceton 6:3/v:v gelöst bzw. suspendiert und die Lösung auf die getrockneten Pellets gesprüht. Freigabewerte in Abhängigkeit von Zeit und pH-Wert:

| Zeit | Medium | pH-Wert | Freigabe in % |
|------|--------|---------|---------------|
| 2 Stunden | künstl. Magensaft | 1,2 | 0 |
| 15 Min. | künstl. Darmsaft | 6,4 | 47,5 |
| 30 Min. | künstl. Darmsaft | 6,4 | 96,9 |

Beispiel 7

AS-E 136 Granulat:

| AS-E 136 | 2,72 kg | (1) |
| Magnesiumhydrogencarbonat | 2,72 kg | (2) |
| Sprengmittel Stärke | 0,95 kg | (3) |
| Polyvinylpyrrolidon | 0,14 kg | (4) |
| Celluloseacetatphthalat | 3,38 kg | (5) |
| Dibutylphthalat | 0,09 kg | (6) |

Die Komponenten (1) bis (4) werden gemischt und mit Hilfe eines Walzenkompaktors brikettiert. Dieses Material wird gebrochen und die Fraktion 0,2-0,45 mm abgesiebt. Feinanteil und Grobanteil wird erneut verdichtet und gebrochen. Die 0,2-0,45 mm großen Teilchen werden mit den Komponenten (5) und (6), gelöst in Äthanol/Methylenchlorid 1:1/v:v, überzogen. Freigabewerte in Abhängigkeit von Zeit und pH-Wert:

| Zeit | Medium | pH-Wert | Freigabe in % |
| --- | --- | --- | --- |
| 1 Stunde | künstl. Magensaft | 1,2 | < 3 |
| 15 Min. | künstl. Darmsaft | 6,0 | 69 |
| 30 Min. | künstl. Darmsaft | 6,0 | 99,3 |

Suspension aus diesem Granulat:

Die überzogenen Teilchen werden anschließend mit den genannten Zusatzstoffen vermischt und in Wasser suspendiert. Die Suspension enthält pro 20 ml:

| magensaftresist. AS-E 136 Granulat | 919,2 mg |
| Zitronensäure | 150,0 mg |
| Saccharose | 5000,0 mg |
| Natrium-Saccharinat | 5,0 mg |
| Orange Aroma | 60,0 mg |
| Xanthan-Gummi | 125,0 mg |

Beispiel 8

AS-E 136 Pellets:

| AS-E 136 | 1,8 kg | (1) |
| Trinatriumphosphat | 1,2 kg | (2) |
| Stearylalkohol | 6,0 kg | (3) |
| Milchzucker | 0,5 kg | (4) |
| Copolymerisat Methacryl-säure-ester (Eudragit S) | 2,2 kg | (5) |
| Hydroxypropylmethylcellulose-phthalat (HP 55) | 0,8 kg | (6) |
| Talcum | 3,0 kg | (7) |

Der Stearylalkohol (3) wird bei 75° C geschmolzen, die Komponenten (1), (2) und (4) werden darin kräftig suspendiert. Die Suspension wird über eine Einstoffphase in einem Kühlturm versprüht. Die sprüherstarrten Teilchen (mehr als 80 % zwischen 0,2 und 0,5 mm Durchmesser) werden mit den Lackkomponenten (5), (6) und (7), gelöst bzw. suspendiert in Isopropanol/Aceton (1:1, v:v) vorsichtig versprüht, um ein Zusammenkleben der feinen Teilchen weitgehend zu vermeiden. Freigabewerte in Abhängigkeit von Zeit und pH-Wert:

| Zeit | Medium | pH-Wert | Freigabe in % |
| --- | --- | --- | --- |
| 30 Min. | künstl. Magensaft | 1,2 | < 3 |
| 15 Min. | künstl. Darmsaft | 6,5 | 74 |
| 30 Min. | künstl. Darmsaft | 6,5 | 99,1 |

## Ansprüche

1. Feste Arzneimittelformen zur oralen Anwendung, enthaltend 9-Deoxo-11-deoxy-9,11-[imino[2-(2-methoxyethoxy)ethyliden]oxy]-(9S)-erythromycin als Wirkstoff, dadurch gekennzeichnet, daß dieser Wirkstoff mit einem basischen Hilfsstoff in einem Verhältnis von 1 Mol Wirkstoff zu mindestens 2 Val basischer Hilfsstoff innig vermischt vorliegt und die aus dieser Mischung und, gegebenenfalls, anderen Hilfsstoffen angefertigten festen Arzneimittelformen mit einem magensaftresistenten Lack, der in einem pH-Bereich zwischen 5,5 und 6,8 den Wirkstoff freigibt, überzogen sind.

2. Feste Arzneimittelformen zur oralen Anwendung gemäß Anspruch 1, dadurch gekennzeichent, daß diese ein Sprengmittel enthalten.

3. Feste Arzneimittelformen zur oralen Anwendung gemäß den Ansprüchen 1 und 2, dadurch gekennzeichnet, daß die innige Mischung aus Wirkstoff und basischem Hilfsstoff zusammen mit üblichen Hilfsstoffen zu Tabletten verpreßt ist und die Tabletten mit einem Lack überzogen sind, der in einem pH-Bereich zwischen 5,5 und 6,8 den Wirkstoff freigibt.

4. Feste Arzneimittelformen zur oralen Anwendung gemäß den Ansprüchen 1 und 2, dadurch gekennzeichnet, daß die innige Mischung aus Wirkstoff und basischem Hilfsstoff zu Pellets geformt vorliegt und die Pellets mit einem Lack überzogen sind, der in einem pH-Bereich zwischen 5,5 und 6,8 den Wirkstoff freigibt.

5. Feste Arzneimittelformen zur oralen Anwendung gemäß den Ansprüchen 1 und 2, dadurch gekennzeichnet, daß die innige Mischung aus Wirkstoff und basischem Hilfsstoff zu Granulaten geformt vorliegt und die Granulate mit einem Lack überzogen sind, der in einem pH-Bereich zwischen 5,5 und 6,8 den Wirkstoff freigibt.

6. Feste Arzneimittelformen zur oralen Anwendung gemäß den Ansprüchen 1 und 2, dadurch gekennzeichnet, daß die innige Mischung aus Wirkstoff und basischem Hilfsstoff in Form von sprüherstarrten sphäroiden Teilchen vorliegt, die mit einem Lack überzogen sind, der in einem pH-Bereich zwischen 5,5 und 6,8 den Wirkstoff freigibt.

7. Feste Arzneimittelformen zur oralen Anwendung gemäß den Ansprüchen 1 bis 6, dadurch gekennzeichent, daß diese mit einem Lack überzogen sind, der in einem pH-Bereich zwischen 6,0 und 6,4 den Wirkstoff freigibt.

8. Feste Arzneimittelformen zur oralen Anwendung gemäß den Ansprüchen 1 bis 7, dadurch gekennzeichnet, daß als basische Hilfsstoffe Magnesiumoxid, Magnesiumhydroxid, Magnesiumcarbcnat, Magnesiumhydrogencarbonat, Calciumhydroxid, Calciumcarbonat, Magnesiumaluminiumhydroxid, Magnesiumaluminat, Natriumcarbonat, Natriumhydrogencarbonat, Kaliumcarbonat, Kaliumhydrogencarbonat, Natrium- oder Kaliumhydroxid, Trinatriumphosphat in einem Verhältnis von 1 mol Wirkstoff zu 2 bis 50 Val basischer Hilfsstoff vorliegen, die Formen mit einem Lack aus Hydroxypropylmethylcellulosephthalat, Copolymerisaten aus Methacrylsäure und -methylester, Celluloseacetatphthalat oder Gemischen dieser Verbindungen mengenmäßig so überzogen sind, daß die Überzüge bis zu 2 Stunden magensaftresistent bleiben und die Mischung aus Wirkstoff und basischem Hilfsstoff noch weitere Hilfsmittel enthält.

9. Feste Arzneimittelformen zur oralen Anwendung gemäß Anspruch 8, dadurch gekennzeichnet, daß als basischer Hilfsstoff eine Mischung verschiedener basischer Hilfsstoffe vorliegt.

10. Verfahren zur Herstellung fester Arzneimittelformen zur oralen Anwendung enthaltend 9-Deoxo-11-deoxy-9,11-[imino[2-(2-methoxyethoxy)ethyliden]oxy]-(9S)-erythromycin als Wirkstoff, dadurch gekennzeichnet, daß dieser Wirkstoff mit einem basischen Hilfsstoff in einem Verhältnis von 1 Mol Wirkstoff zu mindestens 2 Val basischer Hilfsstoff innig vermischt wird und entweder

a) diese innige Mischung nach Zusatz weiterer Hilfsmittel granuliert und zu Tabletten verpreßt oder
b) diese innige Mischung mit einem Klebstoff versehen und pelletiert oder
c) diese innige Mischung kompaktiert, zu einem Granulat aufgebrochen und hieraus eine Fraktion der gewünschten Granulatgröße ausgesiebt oder
d) diese innige Mischung in einer Schmelze suspendiert und die Schmelze in einem Kühlturm zu sphäroiden Teilchen bestimmter Durchmesser versprüht wird und die so erhaltenen Formen anschließend mit einem magensaftresistenten Lacküberzug versehen werden, der den Wirkstoff in einem pH-Bereich zwischen 5,5 und 6,8 freigibt, und, gewünschtenfalls, die nach den Verfahrensschritten c) oder d) erhaltenen Teilchen in einem Suspensionsmittel zur Herstellung eines Sirups suspendiert werden.

## Claims

1. Solid pharmaceutical preparations for oral administration, containing 9-deoxo-11-deoxy-9,11-[imino[2-(2-methoxyethoxy)ethylidene]-oxy]-(9S)-erythromycin as active substance, characterised in that this active substance is intimately mixed with a basic excipient in a ratio of 1 mol of active substance to at least 2 val of basic excipient and the solid pharmaceutical preparations made from this mixture and optionally other excipients are coated with a gastric juice-resistant lacquer which releases the active substance in a pH range of between 5.5 and 6.8.

2. Solid pharmaceutical preparations for oral administration as claimed in claim 1, characterised in that they contain a disintegrant.

3. Solid pharmaceutical preparations for oral administration as claimed in claims 1 and 2, characterised in that the intimate mixture of active substance and basic excipient is compressed to form tablets together with conventional excipients and the tablets are coated with a lacquer which releases the active substance in a pH range of between 5.5 and 6.8.

4. Solid pharmaceutical preparations for oral administration as claimed in claims 1 and 2, characterised in that the intimate mixture of active substance and basic excipient is present in pellet form and the pellets are coated with a lacquer which releases the active substance in a pH range of between 5.5 and 6.8.

5. Solid pharmaceutical preparations for oral administration as claimed in claims 1 and 2, characterised in that the intimate mixture of active substance and basic excipient is present in granulate form and the granules are coated with a lacquer which releases the active substance in a pH range of between 5.5 and 6.8.

6. Solid pharmaceutical preparations for oral administration as claimed in claims 1 and 2, characterised in that the intimate mixture of active substance and basic excipient is present in the form of spray-hardened spheroid particles which are coated with a lacquer which releases the active substance in a pH range of between 5.5 and 6.8.

7. Solid pharmaceutical preparations for oral administration as claimed in claims 1 to 6, characterised in that they are coated with a lacquer which releases the active substance in a pH range of between 6.0 and 6.4.

8. Solid pharmaceutical preparations for oral administration as claimed in claims 1 to 7, characterised in that the basic excipients used are magnesium oxide, magnesium hydroxide, magnesium carbonate, magnesium hydrogen carbonate, calcium hydroxide, calcium carbonate, magnesium aluminium hydroxide, magnesium aluminate, sodium carbonate, sodium hydrogen carbonate, potassium carbonate, potassium hydrogen carbonate, sodium or potassium hydroxide, trisodium phosphate in a ratio of 1 mole of active substance to 2 to 50 val of basic excipient, the preparations are coated with a lacquer consisting of hydroxypropylmethylcellulose phthalate, copolymers of methacrylic acid and methyl methacrylate, cellulose acetate phthalate or mixtures of these compounds in quantities such that the coatings remain resistant to gastric juices for up to 2 hours and the mixture of active substance and basic excipient also contains additional adjuvants.

9. Solid pharmaceutical preparations for oral administration as claimed in claim 8, characterised in that a mixture of different basic excipients is present as the basic excipient.

10. Process for preparing solid pharmaceutical preparations for oral administration containing 9-deoxo-11-deoxy-9,11-[imino-[2-(2-methoxyethoxy)ethylid ene]- oxy]-(9S)-erythromycin as active substance, characterised in that this active substance is intimately mixed with a basic excipient in a ratio of 1 mol of active substance to at least 2 val of basic excipient and either

a) this intimate mixture is granulated, after the addition of further excipients, and compressed to form tablets or

b) this intimate mixture is combined with an adhesive substance and pelleted or

c) this intimate mixture is compacted, broken up into granules and a fraction of the desired granule size is screened out or

d) this intimate mixture is suspended in a melt and the melt is sprayed in a cooling tower to produce spheroid particles of a specific diameter and the preparations thus obtained are subsequently given a coating of a gastric juice-resistant lacquer which releases the active substance in a pH range of between 5.5 and 6.8 and, if desired, the particles obtained according to step c) or d) are suspended in a suspension medium in order to prepare a syrup.

## Revendications

1. Formes galéniques solides pour l'application orale, contenant de la 9-déoxo-11-déoxy-9,11-[imino-[2-(2-méthoxyéthoxy)éthylidène]-oxy]-(9S)-érythromycine comme matière active, caractérisées en ce que cette matière active se présente mélangée intimement avec un adjuvant basique dans un rapport de 1 mole de matière active à au moins 2 val d'adjuvant basique et en ce que les formes galéniques solides préparées à partir de ce mélange et, éventuellement, d'autres adjuvants sont enrobées d'un vernis résistant au suc gastrique qui libère la matière active dans un domaine de pH compris entre 5,5 et 6,8.

2. Formes galéniques solides pour l'application orale selon la revendication 1, caractérisées en ce qu'elles contiennent un désagrégeant.

3. Formes galéniques solides pour l'application orale selon les revendications 1 et 2, caractérisées en ce que le mélange intime de la matière active et de l'adjuvant basique, ensemble avec des adjuvants usuels, est pressé en comprimés et en ce que les comprimés sont enrobés d'un vernis qui libère la matière active dans un domaine de pH compris entre 5,5 et 6,8.

4. Formes galéniques solides pour l'application orale selon les revendications 1 et 2, caractérisées en ce que le mélange intime de la matière active et de l'adjuvant basique se présente formé en boulettes et en ce que les boulettes sont enrobées d'un vernis qui libère la matière active dans un domaine de pH compris entre 5,5 et 6,8.

5. Formes galéniques solides pour l'application orale selon les revendications 1 et 2, caractérisées en ce que le mélange intime de la matière active et de l'adjuvant basique se présente formé en granulés et en ce que les granulés sont enrobés d'un vernis qui libère la matière active dans un domaine de pH compris entre 5,5 et 6,8.

6. Formes galéniques solides pour l'application orale selon les revendications 1 et 2, caractérisées en ce que le mélange intime de la matière active et de l'adjuvant basique se présente sous forme de particules sphéroïdes durcies par pulvérisation, qui sont enrobées d'un vernis qui libère la matière active dans un domaine de pH compris entre 5,5 et 6,8.

7. Formes galéniques solides pour l'application orale selon les revendications 1 à 6, caractérisées en ce qu'elles sont enrobées d'un vernis qui libère la matière active dans un domaine de pH compris entre 6,0 et 6,4.

8. Formes galéniques solides pour l'application orale selon les revendications 1 à 7, caractérisées en ce que de l'oxyde de magnésium, de l'hydroxyde de magnésium, du carbonate de magnésium, du bicarbonate de magnésium, de l'hydroxyde de calcium, du carbonate de calcium, de l'hydroxyde de magnésium et d'aluminium, de l'aluminate de magnésium, du carbonate de sodium, du bicarbonate de sodium, du carbonate de potassium, du bicarbonate de potassium, de l'hydroxyde de sodium ou de potassium, du phosphate trisodique en tant qu'adjuvants basiques se présentent dans un rapport de 1 mole de matière active à 2 jusqu'à 50 val d'adjuvant basique, en ce que les formes sont enrobées d'un vernis constitué de phtalate d'hydroxypropylméthycellulose, de copolymères d'acide méthacrylique et de méthacrylate de méthyle, d'acétophatalate de cellulose ou de mélanges de ces composés, en des quantités telles que les enrobages restent résistants au suc gastrique jusqu'à 2 heures et en ce que le mélange de la matière active et de l'adjuvant basique contient encore d'autres adjuvants.

9. Formes galéniques solides pour l'application orale selon la revendication 8, caractérisées en ce qu'il y a, en tant qu'adjuvant basique, un mélange de différents adjuvants basiques.

10. Procédé de préparation de formes galéniques solides pour l'application orale contenant de la 9-déoxo-11-déoxy-9,11-[imino-[2-(2-méthoxyéthoxy) éthylidène]oxy]-(9S)-érythromycine comme matière active, caractérisé en ce que cette matière active est mélangée intimement avec un adjuvant basique dans un rapport de 1 mole de matière active à au moins 2 val d'adjuvant basique et soit
   a) ce mélange intime est granulé et pressé en comprimés, après addition d'autres adjuvants ou
   b) ce mélange intime est muni d'un agglutinant et transformé en boulettes ou
   c) ce mélange intime est compacté, brisé en granulés et en ce qu'on en sépare par tamisage une fraction de la taille de granulés souhaitée ou
   d) ce mélange intime est mis en suspension dans une masse fondue et la masse fondue est pulvérisée dans une tour de refroidissement pour donner des particules sphéroïdes de diamètre déterminé et en ce que les formes ainsi obtenues sont ensuite munies d'un enrobage de vernis résistant au suc gastrique, lequel enrobage libère la matière active dans un domaine de pH compris entre 5,5 et 6,8, et, si on le souhaite, les particules obtenues selon les étapes de procédé c) ou d) sont mises en suspension dans un adjuvant de suspension pour la préparation d'un sirop.